# FASCICULE DE BREVET EUROPEEN

(11) **EP 2 483 226 B1**
(45) Date de publication et mention de la délivrance du brevet: **26.07.2017**
(21) Numéro de dépôt: 10759917.7
(22) Date de dépôt: 30.09.2010
(51) Int. Cl.: C07C 41/36, C07C 51/47, C07C 59/64, C07C 43/295, C07C 45/67, C07C 47/58, C07C 47/575

(54) **PROCEDE DE SEPARATION DE COMPOSES PHENOLIQUES SOUS FORME SALIFIEE**
VERFAHREN ZUR ABSCHEIDUNG VON VERSALZTEN PHENOLVERBINDUNGEN
METHOD FOR SEPARATING SALIFIED PHENOLIC COMPOUNDS

(30) Priorité: 02.10.2009 FR 0904701
(43) Date de publication de la demande: 08.08.2012
(73) Titulaire: Rhodia Operations, 75009 Paris (FR)
(72) Inventeur: DESOUHANT-MASSACRET, Magali, F-69300 Caluire (FR)
(74) Mandataire: Menville, Laure
(86) Numéro de dépôt international: PCT/EP2010/064602
(87) Numéro de publication internationale: WO 2011/039331

(56) Documents cités:
- FR-A1- 2 931 476
- US-B1- 6 359 172

## Description

La présente invention a pour objet un procédé de séparation de composés phénoliques sous forme salifiée, à partir d'un milieu réactionnel les comprenant.

Plus précisément, l'invention se rapporte à la séparation du gaïacol ou du guétol sous forme salifiée, à partir des milieux de synthèse les comprenant.

L'invention vise plus particulièrement la récupération du gaïacol sous forme de sel de sodium présent en quantité excédentaire au cours de la synthèse de la vanilline ou 4-hydroxy-3-méthoxybenzaldéhyde.

Les aldéhydes hydroxy- et alkoxyaromatiques sont des produits très importants utilisés comme arômes et parfums et comme produits intermédiaires dans de nombreux domaines tels que par exemple, l'agrochimie, la pharmacie, la cosmétique et autres industries.

Les ortho- et para-hydroxybenzaldéhydes, le 4-hydroxy-3-méthoxybenzaldéhyde et le 3-éthoxy-4-hydroxybenzaldéhyde dénommés respectivement « vanilline » et « éthylvanilline » sont parmi les produits les plus importants.

II a été proposé différents procédés pour la synthèse des aldéhydes aromatiques.

Les procédés les plus importants sont basés sur la fonctionnalisation d'un composé phénolique au départ, phénol, dérivé du catéchol, gaïacol (ou 2-méthoxyphénol), guétol (ou 2-éthoxyphénol).

Intervient, généralement, dans ce type de procédé, le composé phénolique sous une forme salifiée, par exemple, sous forme d'un sel de sodium.

Ainsi, par exemple, de nombreux procédés de préparation de la vanilline font intervenir un sel du gaïacol comme substrat sur lequel est ensuite additionné un groupe formyle en position para du groupe hydroxyle, selon différentes méthodes.

Une voie d'accès classique à la vanilline implique une réaction de condensation de l'acide glyoxylique sur le gaïacol, en milieu basique, pour obtenir l'acide 4-hydroxy-3-méthoxymandélique. Ce produit est ensuite oxydé pour conduire à la vanilline.

La réaction est habituellement conduite en présence de soude et avec un excès de gaïacol : l'acide glyoxylique étant le réactif déficitaire.

Ainsi, en fin de réaction de condensation, on obtient un mélange réactionnel aqueux comprenant le sel de sodium de l'acide 4-hydroxy-3-méthoxymandélique, précurseur de la vanilline, des produits secondaires comme les sels de sodium des acide 2-hydroxy-3-méthoxymandélique et acide 4-hydroxy-5-méthoxy-1,3-dimandélique et un excès plus ou moins important de gaïacolate de sodium.

Ainsi, dans ce milieu réactionnel, il y a présence de plusieurs types de composés phénoliques salifiés à savoir le gaïacol en excès sous forme de gaïacolate de sodium et les produits de la réaction qui sont également des composés phénoliques salifiés tels que les sels de sodium des acides 4-hydroxy-3-méthoxymandélique, 2-hydroxy-3-méthoxymandélique et 4-hydroxy-5-méthoxy-1,3-dimandélique.

Pour des considérations économiques, il importe de récupérer le substrat de départ qui n'a pas réagi. Toutefois, l'opération n'est pas aisée car le gaïacol est sous forme de gaïacolate de sodium et est en présence de composés phénoliques également salifiés et de structure proche.

Dans certains procédés décrits dans l'état de la technique, en particulier dans FR 2 132 364, le gaïacolate de sodium en fin de réaction de condensation est transformé en gaïacol par un traitement acide, le plus souvent l'acide sulfurique.

On extrait alors le gaïacol non converti de la solution acide par un traitement d'extraction à l'aide d'un hydrocarbure, par exemple le benzène ou le toluène.

L'inconvénient d'un tel procédé est de faire appel à un solvant organique ce qui induit des opérations supplémentaires de distillation afin de pouvoir recycler le solvant organique et le substrat récupéré. De plus, au cours de la distillation, il y a des réactions secondaires conduisant à la formation de lourds.

Par ailleurs, la neutralisation du gaïacolate de sodium par l'acide sulfurique génère du sulfate de sodium ce qui entraîne la formation d'effluents salins importants.

De plus, le gaïacol récupéré doit être à nouveau salifié pour être introduit dans la réaction de condensation avec l'acide glyoxylique.

De même, le milieu réactionnel comprenant les composés mandéliques avec un groupe hydroxyle libre, doit être à nouveau salifié pour être introduit dans la réaction d'oxydation permettant d'obtenir la vanilline. Le document US 6,359,172 divulgue un procédé de séparation des composants d'une mixture contenant du phénol et des dérivés d'acide hydroxymandélique utilisant une résine échangeuse d'anions comportant des fonctions basiques. Les dérivés mandéliques sont adsorbés sur la résine alors que le phénol est élué. Pour pallier ces inconvénients, l'invention propose un procédé permettant de récupérer l'excès de composé phénolique de départ sous forme salifiée, en particulier le gaïacolate de sodium selon un procédé qui ne fait pas intervenir cette étape de neutralisation du gaïacolate de sodium en gaïacol, ledit gaïacol devant être extrait à l'aide d'un solvant organique qu'il faut ensuite séparer par une distillation.

Il a maintenant été trouvé et c'est ce qui constitue l'objet de la présente invention, un procédé de séparation de composés phénoliques sous forme salifiée à partir d'un milieu réactionnel aqueux résultant de la réaction d'un composé phénolique et de l'acide glyoxylique en présence d'une base conduisant à un milieu réactionnel comprenant au moins l'excès de composé phénolique de départ sous forme salifiée et les différents composés mandéliques sous forme salifiée résultant de la réaction, caractérisé par le fait que l'on met en contact ledit milieu réactionnel comprenant le composé phénolique de départ sous forme salifiée avec un support adsorbant conduisant à l'adsorption sélective dudit composé phénolique sur ledit support et à la récupération d'un flux aqueux comprenant les composés mandéliques sous forme salifiée issus de la réaction et que l'on désorbe le composé phénolique fixé sur l'adsorbant, par un traitement de régénération dudit adsorbant.

Dans l'exposé qui suit de la présente invention, on entend "par composé phénolique de départ", un composé benzénique dont au moins un atome d'hydrogène directement lié au noyau benzénique est substitué par un groupe hydroxyle.

Conformément au procédé de l'invention, il a été trouvé dans le cas du traitement d'un milieu réactionnel aqueux comprenant du gaïacolate de sodium, que celui-ci pouvait s'adsorber sur le support adsorbant ce qui permettait de le séparer et ensuite, après désorption, de le recycler à l'étape de synthèse sans être obligé de passer par une étape d'acidification du gaïacolate de sodium pour le transformer en gaïacol qui est récupéré puis ensuite soumis à un nouveau traitement basique car c'est un phénolate qui est mis en oeuvre à l'étape de condensation.

Afin d'illustrer le procédé de l'invention, la Demanderesse cite le cas de la séparation du gaïacolate de sodium à partir du milieu aqueux de condensation du gaïacolate de sodium et de l'acide glyoxylique.

Toutefois, le procédé de l'invention n'est pas limité à la séparation de ce substrat et convient également pour des composés phénoliques de départ sous forme salifiée répondant à la formule suivante : 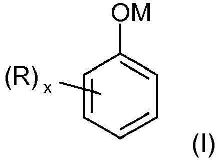 dans ladite formule :
- R est un groupe alkyle ou alkoxy ayant de 1 à 4 atomes de carbone, un atome d'halogène,
- x est un nombre allant de 0 à 3, et plus préférentiellement égal à 1,
- M représente un cation d'un élément métallique du groupe (IA) de la classification périodique à savoir le lithium, sodium, potassium, rubidium et césium ou un cation ammonium.

Dans la formule (I), M est de préférence le sodium.

Comme exemples de groupes alkyle, on peut citer les groupes alkyle, linéaires ou ramifiés ayant de 1 à 4 atomes de carbone, tels que méthyle, éthyle, n-propyle, isopropyle, n-butyle, isobutyle, sec-butyle, tert-butyle. Parmi ceux-ci, les groupes méthyle et éthyle sont préférés.

Comme exemples de groupes alkoxy linéaires ou ramifiés ayant de 1 à 4 atomes de carbone, on peut mentionner les groupes méthoxy, éthoxy, n-propoxy, isopropoxy, n-butoxy, isobutoxy, sec-butoxy. Les groupes méthoxy et éthoxy sont préférés.

R représente également un atome d'halogène, de préférence fluor, chlore et brome et plus préférentiellement fluor.

Pour ce qui est de la nature du R, il est à noter que la liste des substituants donnée n'est pas limitative et d'autres substituants peuvent être envisagés dans la mesure où ceux-ci ne gênent pas la séparation du composé de formule (I).

A titre illustratif de composés répondant à la formule (I), on peut mentionner plus particulièrement les sels des composés choisis dans le groupe constitué par : le phénol , le gaïacol, le 3-méthoxyphénol, le guétol, le 3-éthoxyphénol, le 2-isopropoxyphénol, le 3-isopropoxyphénol, le 2-méthoxy-5-méthylphénol, le 2-méthoxy-6-méthylphénol, le 2-méthoxy-6-tert-butylphénol, le 3-chloro-5-méthoxyphénol, le 2,3-diméthoxy-5-méthylphénol, le 2,3-diméthoxyphénol, le 2,6-diméthoxyphénol, le 3,5-diméthoxyphénol, les crésols, le tert-butylphénol, le 2-méthoxyphénol, le 4-méthoxyphénol.

Les composés de formule (I) préférés sont le phénol, le gaïacol, le guétol.

Selon l'invention, on applique le procédé de l'invention au milieu réactionnel aqueux issu de la réaction d'un composé phénolique sous forme salifiée de formule (I) et de l'acide glyoxylique.

La réaction de condensation du composé phénolique de formule (I) et de l'acide glyoxylique peut être conduite en présence d'un hydroxyde d'ammonium mais plus préférentiellement en présence d'un hydroxyde de métal alcalin qui peut être l'hydroxyde de sodium ou de potassium. Pour des considérations économiques, on choisit de préférence l'hydroxyde de sodium.

Pour ce qui est de l'acide glyoxylique, on fait appel à une solution aqueuse d'acide glyoxylique ayant une concentration variant par exemple, entre 15 et 70 % en poids.

On fait réagir l'acide glyoxylique sur le composé phénolique de formule (I) en excès. Le rapport molaire entre le composé phénolique de formule (I) et l'acide glyoxylique varie entre 1,1 et 4,0, de préférence entre 1,5 et 3,0.

La solution d'hydroxyde de métal alcalin mise en oeuvre a une concentration généralement comprise entre 10 et 50 % en poids.

La quantité d'hydroxyde de métal alcalin introduite dans le milieu réactionnel tient compte de la quantité nécessaire pour salifier la fonction hydroxyle du composé phénolique de formule (I) et de la quantité nécessaire pour salifier la fonction carboxylique de l'acide glyoxylique.

La concentration du composé phénolique de formule (I) est comprise de préférence entre 0,5 et 1,5 moles/litre.

La température de la réaction est choisie avantageusement entre 20°C et 60°C.

La réaction est conduite à pression atmosphérique mais sous atmosphère contrôlée de gaz inertes, de préférence d'azote ou de gaz rares, en particulier l'argon. On choisit préférentiellement l'azote.

Après mise en contact du composé phénolique de formule (I), de l'acide glyoxylique et de l'hydroxyde de métal alcalin, on maintient le milieu réactionnel sous agitation et à la température choisie dans l'intervalle précité pendant une durée variable allant de 1 à 10 heures.

En fin de réaction, on obtient un milieu réactionnel aqueux comprenant l'excès de composé phénolique sous forme salifiée répondant à la formule (I) et différents composés mandéliques sous forme salifiée désignés par l'expression « composés mandéliques » et répondant aux formules suivantes : 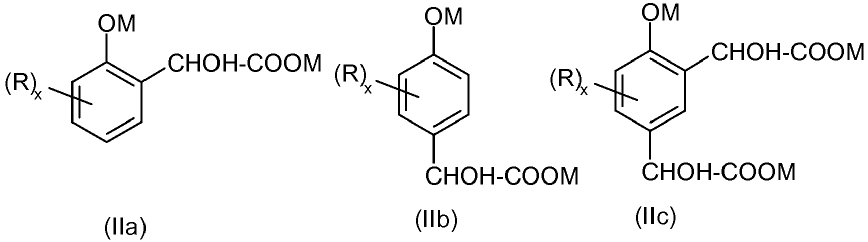 dans lesdites formules, M, R et x ont la signification donnée pour la formule (I).

Les composés mandéliques préférés répondent aux formules (IIa), (IIb) et (IIc) dans lesquelles M représente un atome de sodium, x est un nombre allant de 0 à 3, de préférence égal à 1 et les groupes R, identiques ou différents, représentent un groupe alkyle ou alkoxy ayant de 1 à 4 atomes de carbone, de préférence un groupe méthoxy ou éthoxy ou un atome d'halogène.

L'invention s'applique plus particulièrement dans le cadre de la préparation de la vanilline à un milieu aqueux comprenant du gaïacolate de sodium et des composés mandéliques sous forme salifiée : acides 4-hydroxy-3-méthoxymandélique, 2-hydroxy-3-méthoxymandélique, 4-hydroxy-5-méthoxy-1,3-dimandélique sous forme salifiée.

L'invention s'applique également préférentiellement à la préparation de l'éthylvanilline à un milieu réactionnel qui est un milieu aqueux comprenant du guétolate de sodium et des composés mandéliques sous forme salifiée : acides 3-éthoxy-4-hydroxymandélique, 3-éthoxy-2-hydroxymandélique, 5-éthoxy-4-hydroxy-1,3-dimandélique sous forme salifiée.

La concentration pondérale du composé phénolique de départ sous forme salifiée, de préférence le gaïacolate ou le guétolate de sodium varie généralement entre 1 % et 20 % en poids, de préférence entre 5 % et 10 % en poids.

La concentration pondérale de composés mandéliques (o- p- et dimandélate) dans le milieu réactionnel est habituellement comprise entre 3 % et 30 % en poids, de préférence entre 5 % et 20 % en poids.

Le procédé de l'invention de séparation du composé phénolique sous forme salifiée, est donc mis en oeuvre sur le milieu réactionnel aqueux tel que précédemment défini.

Dans le cas de la préparation de la vanilline et de l'éthylvanilline, il comprend donc du gaïacolate de sodium ou du guétolate de sodium et différents produits de réaction à savoir des mandélates de sodium, mono- ou difonctionnels tels que précisés selon les formules (IIa), (IIb) et (IIc) avec prépondérance du composé de formule (IIb).

Conformément au procédé de l'invention, on met en contact le milieu réactionnel tel que précédemment décrit avec un support adsorbant conduisant à l'adsorption sélective sur le support du composé phénolique et à la récupération d'un flux aqueux comprenant les différents composés mandéliques puis l'on désorbe le composé phénolique par régénération du support adsorbant à l'aide d'un moyen approprié de préférence à l'aide d'une base.

On entend par « support adsorbant » un support solide capable d'adsorber sur sa surface, les molécules d'un substrat appelé « adsorbat » par liaison de Van Der Waals.

Dans la suite du texte, on désignera d'une manière simplifiée, le support adsorbant par « adsorbant ».

Les adsorbants sont généralement des supports ayant une très grande surface spécifique et présentant une porosité interne.

Les surfaces spécifiques indiquées font référence à une surface spécifique déterminée selon la méthode **B**RUNEAU-**E**MMETT-**T**ELLER décrite dans le périodique « The Journal of American Society 60,309 (1938) ».

Le choix de l'adsorbant doit tenir compte de plusieurs impératifs.

L'adsorbant doit être physiquement et chimiquement stable vis-à-vis des conditions opératoires.

L'adsorbant doit avoir une bonne capacité d'adsorber l'adsorbat.

L'efficacité de l'adsorption se mesure en pourcentage de l'adsorbat par rapport à la masse de l'adsorbant. Elle se situe généralement entre 10 et 50 % en poids.

L'adsorbant doit présenter une bonne sélectivité d'adsorption du composé phénolique par rapport aux composés mandéliques.

L'adsorbant doit être facilement régénérable c'est-à-dire que l'adsorbat doit être aisément désorbé du support adsorbant.

Comme adsorbants susceptibles d'être utilisés dans le procédé de l'invention, on peut citer notamment les charbons actifs, les polymères adsorbants, les zéolithes et les tamis moléculaires.

L'adsorbant utilisé selon la présente invention peut être un charbon activé.

Il existe de nombreuses qualités de charbons actifs et l'on peut faire appel aux charbons dit "physiques" qui résultent d'une étape de calcination à haute température d'une matière première carbonée suivie généralement par une étape d'activation thermique qui consiste à augmenter son pouvoir adsorbant.

Le charbon actif peut être à base de houille, de tourbe, de lignite, de résidus de distillation du pétrole, ou à partir de toute matière organique végétale riche en carbone : bois, écorces, brindilles, pâte de bois, coques de fruits notamment coques de noix de coco, coques de cacahuètes.

Les traitements post-calcination sont destinés à éliminer les produits (minéraux, goudrons) qui obstruent les pores.

Les traitements post-combustions peuvent être une activation physique qui consiste en une nouvelle combustion à haute température effectuée dans un courant d'air et de vapeur d'eau, injectés sous pression, qui va créer une porosité à pores étroits sur la surface du charbon, augmentant de façon très importante sa surface et son pouvoir d'adsorption.

L'activation peut être également chimique effectuée par exemple à l'aide d'acide nitrique ou phosphorique conduisant à un charbon actif à pores plus larges.

Le diamètre des pores dépend également des pores existant dans la matière première utilisée. Les coques de noix de coco et de bois très denses donnent des micropores (< 2 nm), les bois moyens à blanc donne des mésopores (entre 2 et 50 nm) ou des macropores (> 50 nm).

La surface développée par le charbon actif est énorme : un gramme de charbon actif a une surface spécifique comprise entre 400 et 2 500 m²/g, de préférence entre 500 et 1000 m²/g.

L'indice d'iode qui définit le nombre de mg d'iode adsorbé par g de charbon actif (ASTM D4607-94) est le plus souvent supérieur ou égal à 1000 et est généralement compris entre 1000 et 1300.

Les charbons actifs peuvent être mis en oeuvre sous forme de granulés notamment d'extrudés ayant une taille allant par exemple de 0,4 à 2 mm, de préférence de 0,5 à 1,5 mm (ASTM D2862-97).

L'invention n'exclut pas la mise en oeuvre d'adsorbants carbonés formés par exemple, par pyrolyse de résines polymères.

Des adsorbants convenant à l'invention sont obtenus par pyrolyse de résine échangeuse d'ions macroréticulée styrène sulfonés / divinylbenzène.

On peut se référer notamment au brevet américain 5 094 754. De tels adsorbants sont commercialisés par Rohm and Haas Company, sous la marque déposée Ambersorb, par exemple Ambersorb 563.

Un autre type d'adsorbants convenant au procédé de l'invention, sont les polymères adsorbants.

Les polymères adsorbants se présentent généralement sous forme de billes ayant une structure poreuse.

Les polymères de base sont des polystyrènes, notamment des copolymères styrène-divinylbenzène, des polyacryliques en particulier des copolymères divinylbenzène - ester acrylique ou des polymères phénoliques, notamment des copolymères phénol/formaldéhyde.

La porosité est créée par une forte réticulation du polymère.

Les adsorbants polymériques présentent généralement une surface spécifique variant de 100 à 1000 m²/g, de préférence comprise entre 400 et 800 m²/g_{.}

Ce sont généralement des matériaux mésoporeux ayant une taille de pores s'échelonnant de 4 à 60 nm : leur porosité interne se situant entre 0,4 et 1,2 cm³/g_{.}

Les adsorbants préférés sont des adsorbants inertes ne comprenant pas de groupes fonctionnels et plus particulièrement les copolymères mésoporeux de styrène et de divinylbenzène à forte réticulation et à forte porosité.

Conviennent plus particulièrement à la mise en oeuvre du procédé de l'invention, les polymères adsorbants mésoporeux de styrène et de divinylbenzène commercialisés par la Société Rohm et Haas sous la dénomination commerciale Amberlite XAD et plus préférentiellement les polymères suivants : Amberlite XAD FPX66 ; Amberlite XAD 4 ; Amberlite XAD 16 ; Amberlite XAD 761 ; Amberlite XAD 1180N ; Amberlite XAD 1600N ; Amberlite XAD 18 ; Amberlite XAD7HP.

Comme autres polymères adsorbants, on peut mentionner ceux commercialisés sous la dénomination PUROLITE Hypersol Macronet, PUROLITE Purosorb ainsi que ainsi que ceux vendus par la Société Lanxess et plus particulièrement les LEWATIT VP OC 1064 MD PH et LEWATIT VP OC 1163.

Comme autres adsorbants, on peut également mentionner les zéolithes.

Par "zéolithe", on entend un tectosilicate cristallisé d'origine naturelle ou synthétique dont les cristaux résultent de l'assemblage tridimensionnel d'unités tétraédriques de SiO₄ et TO₄ : T représentant un élément trivalent tel que aluminium, gallium, bore, fer, de préférence, l'aluminium.

Les zéolithes de type aluminosilicate sont les plus communes.

Les zéolithes présentent au sein du réseau cristallin, un système de cavités reliées entre elles par des canaux d'un diamètre bien défini que l'on appelle les pores et les canaux s'assemblent en un réseau monodimensionnel, bidimensionnel ou tridimensionnel.

Dans le procédé de l'invention, on fait appel préférentiellement à des zéolithes à réseau bidimensionnel ou tridimensionnel.

Bien que l'on puisse mettre en oeuvre une zéolithe naturelle, on préfère choisir une zéolithe synthétique qui présente des caractéristiques physico-chimiques constantes.

On choisit avantageusement une zéolithe ayant un diamètre moyen de pores supérieur ou égal à 6 , et de préférence compris entre 6 et 8 Å.

Comme exemples de zéolithes convenant particulièrement pour la mise en oeuvre du procédé de l'invention, on peut citer les zéolithes à réseau bidimensionnel notamment les zéolithes de type mordénite ; les zéolithes à réseau tridimensionnel notamment les zéolithes de type β, les zéolithes de type faujasite, en particulier les zéolithes Y ou les zéolithes mésoporeuses de type MCM.

On précisera, à titre indicatif, que le diamètre moyen des pores des zéolithes mordénites, β et Y sont respectivement de l'ordre de 6,5 Å, 6,8 Å et 7,2 Å.

Dans les différentes zéolithes, le rapport Si/Al peut varier largement.

Les mordénites ont un rapport molaire Si/Al de 5 à 50.

Les zéolithes β ont un rapport molaire Si/Al supérieur à 8, de préférence, compris entre 10 et 100, et encore plus préférentiellement entre 12 et 50,

Les zéolithes Y en particulier les zéolithes obtenues après traitement de désalumination (par exemple hydrotraitement, lavage à l'aide d'acide chlorhydrique ou traitement par SiCl₄) ont des rapports molaires Si/Al supérieur à 3, de préférence compris entre 6 et 100 ;

Les zéolithes mésoporeuses de type MCM, plus particulièrement MCM-41 et MCM-48 ont des rapports molaires Si/Al compris entre 10 et 100, de préférence, compris entre 15 et 40. Elles présentent une surface spécifique BET comprise entre 700 et 1000 m²/g, un diamètre des pores, variant généralement entre 15 et 40 Å.

Parmi toutes ces zéolithes, on fait appel préférentiellement dans le procédé de l'invention aux zéolithes β et Y.

Les zéolithes mises en oeuvre dans le procédé de l'invention, sont des produits connus décrits dans la littérature [cf. Atlas of zeolites structure types by W. M. Meier and D. H. Oison published by the Structure Commission of the International Zeolite Association (1978)].

On peut faire appel aux zéolithes disponibles dans le commerce ou bien les synthétiser selon les procédés décrits dans la littérature notamment selon les références mentionnées dans l'Atlas précité.

Conviennent également au procédé de l'invention, les tamis moléculaires.

Les tamis moléculaires sont des zéolithes synthétiques qui se caractérisent par une structure cristalline et un diamètre de pore régulier.

Ce sont des alumino-silicates métalliques qui possèdent une structure cristalline tridimensionnelle constituée par un assemblage de tétraèdres SiO₄ et AlO₄ et qui contiennent des cations tels que Na⁺, K⁺ ou Ca⁺⁺ pour rendre le système électriquement neutre.

Les tétraèdres sont assemblés de telle manière qu'ils composent un octaèdre tronqué. Ces octaèdres sont eux mêmes arrangés selon une structure cristalline cubique simple, formant un réseau dont les cavités qui ont un diamètre approximatif de 11,5 Å, sont accessibles par des ouvertures ou pores.

La famille des tamis moléculaires communément appelée MS comporte les tamis moléculaires 3A, 4A et 5A.

Les tamis moléculaires de type A sont caractérisés par un rapport molaire Si/AI voisin de 1.

La taille d'ouverture de pores varie selon le type de tamis moléculaires car les pores peuvent être bloqués au moyen de cations et par échange de cations, il devient possible de modifier la taille des pores.

Lorsque ces cations sont dérivés du sodium, les pores ont un diamètre d'ouverture de 4,1Å, et l'on a alors un tamis moléculaire dit 4A.

Le tamis moléculaire 4A est obtenu par échange d'une grande partie des ions sodium par des ions potassium, le diamètre des pores étant d'environ 3Å.

Le tamis moléculaire 5A est réalisé en remplaçant les ions sodium par des ions calcium, le diamètre des pores étant alors de l'ordre de 5Å.

Les tamis de type 3A, 4A ou 5A sont disponibles commercialement, sous forme de poudre et éventuellement sous forme de compositions avec d'autres substances notamment un liant argileux qui peuvent se présenter sous forme de granulés, billes ou extrudés.

Parmi les différents tamis précités, il y a lieu de faire intervenir préférentiellement dans le procédé de l'invention, le tamis 5A.

Conformément au procédé de l'invention, on fait passer le milieu réactionnel obtenu en fin de réaction de condensation sur le support adsorbant.

Le flux est ainsi à une température voisine de la température de condensation comprise entre 20°C et 60°C.

Généralement, le support adsorbant est placé dans un réacteur agité ou bien dans une colonne : le milieu étant introduit généralement de haut en bas.

La quantité de support adsorbant mise en oeuvre est déterminée en fonction de l'efficacité d'adsorption du support adsorbant.

Comme mentionné précédemment, l'efficacité d'adsorption varie généralement entre 10 et 50 %. Ainsi, afin d'avoir une adsorption complète, on adapte la quantité d'adsorbant mise en oeuvre qui sera d'autant plus élevée que l'efficacité d'adsorption est faible.

On précisera que la quantité d'adsorbant représente au moins de 2 à 10 fois le poids du composé phénolique sous forme salifiée à adsorber. On met en oeuvre de préférence un excès d'adsorbant, par exemple un excès de 10 à 20 % du poids d'adsorbant calculé.

On récupère en pied de colonne un flux aqueux comprenant tous les composés mandéliques sous forme salifiée alors que le composé phénolique est adsorbé sur le support.

Le flux aqueux comprenant les composés mandéliques sous forme salifiée peut être directement engagé à l'opération d'oxydation permettant d'obtenir l'aldéhyde aromatique correspondant aux composés mandéliques sous forme salifiée.

Dans une étape suivante, on récupère le composé phénolique sous forme salifiée, par régénération de l'adsorbant.

On choisit de préférence de régénérer l'adsorbant à l'aide d'une base.

Comme bases convenant, on peut citer notamment, la soude ou la potasse.

A cet effet, on effectue un traitement basique, de préférence à l'aide d'une solution aqueuse basique, ayant une concentration de 1 à 10 % en poids, et plus préférentiellement entre 2 et 8 % en poids. La soude est habituellement utilisée.

La quantité de base mise en oeuvre est au moins égale à la quantité de composé phénolique sous forme salifiée à régénérer.

On obtient alors une solution du composé phénolique qui est salifié et donc directement recyclable à l'étape de condensation.

Comme mentionné précédemment, le procédé de l'invention permet de séparer un flux aqueux comprenant les différents composés mandéliques sous forme salifiée.

Ainsi, le procédé de l'invention permet d'accéder aux aldéhydes hydroxyaromatiques correspondant aux formules (IIa), (IIb) et (IIc) dans lesquelles le groupe glycolique de formule -CHOH-COOH est remplacé par un groupe formyle CHO.

La réaction d'oxydation peut être conduite selon les techniques décrites dans la littérature. Ainsi, on peut utiliser les catalyseurs classiquement utilisés dans les réactions d'oxydation des composés mandéliques, en milieu basique.

L'oxydation est généralement conduite par l'oxygène ou l'air sous pression, en présence d'un catalyseur approprié tel que par exemple, les dérivés du chrome, manganèse, fer, cobalt, nickel, cuivre, zinc, bismuth, aluminium, argent, vanadium ou osmium.

Il est à noter que cette liste n'est pas limitative.

On peut mettre en oeuvre tout particulièrement les oxydes, sulfates, halogénures, acétates desdits éléments métalliques.

On peut également utiliser un catalyseur comprenant au moins deux éléments métalliques. On peut se référer plus particulièrement à WO 2008/148760 qui propose la mise en oeuvre d'un système catalytique comprenant au moins deux éléments métalliques M₁ et M₂ choisis dans le groupe formé par : le cuivre, le nickel, le cobalt, le fer et le manganèse.

Ainsi, l'invention permet d'accéder facilement aux hydroxybenzaldéhydes et plus particulièrement à la vanilline et ses analogues, par exemple 3-éthylvanilline, 3-isopropylvanilline, par oxydation respectivement de l'acide p-hydroxymandélique et des acides 4-hydroxy-3-méthoxymandélique, 3-éthoxy-4-hydroxy-mandélique, ou 4-hydroxy-3-isopropoxymandélique.

On donne ci-après, des exemples de réalisation de l'invention donnés à titre illustratif et sans caractère limitatif.

Dans les exemples, la sélectivité de la réaction est définie comme le rapport molaire [gaiacol]adsorbé/([gaiacol]adsorbé+[mandélate]adsorbé).

### Exemple 1

Dans cet exemple, l'adsorbant utilisé est le tamis moléculaire 5A.

Ce solide est un aluminosilicate de calcium. Il possède un diamètre de pores de 0,5 nm.

Dans un tube shott, on charge 20 g de flux comprenant 0,95 g de gaïacolate de sodium (0,0065 mol) et 1,32 g de p-mandélate de sodium (0,0055 mol) de formule :

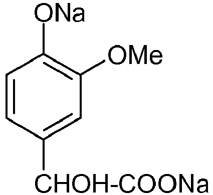

On ajoute ensuite 2 g de tamis moléculaire 5A et le milieu réactionnel est agité à 40°C pendant toute la durée de l'adsorption.

L'adsorption est suivie par analyse chromatographie liquide haute performance de ladite solution.

Au bout de 15 min, 45 % de gaïacolate sont adsorbés.

Au bout de 1 h 30, on adsorbe 55 % en poids de gaïacolate (exprimés par rapport au poids total de gaïacolate présent dans la solution à traiter), sans fixer de mandélates.

Le rapport qui définit la sélectivité est de 1.

### Exemple 2

Dans cet exemple, on met en oeuvre une zéolithe Y à savoir la zéolithe hydrophobe Wessalith DAY 55. Le diamètre de pores de cette zéolithe est de 0,72 nm.

On reproduit l'exemple 1 à la seule différence que la nature de l'adsorbant est changée.

Avec cet adsorbant, 44 % de gaïacolate sont adsorbés au bout de 15 min sans adsorber de mandélates.

La sélectivité obtenue est de 1.

### Exemple 3

Dans cet exemple, on met en oeuvre un polymère adsorbant dénommé Amberlite XAD16, copolymère de styrène et de divinylbenzène qui est un polymère non ionique. Il se présente sous forme de billes, possède une grande surface spécifique supérieure à 700 m²/g et sa structure est très poreuse : la taille moyenne des pores étant de 10 nm.

On reproduit l'exemple 1 à la seule différence que la nature de l'adsorbant est changée.

Avec cet adsorbant, la sélectivité obtenue est de 1 et il reste en solution 60 % de gaïacolate.

### Exemple 4

Dans cet exemple, on met en oeuvre comme adsorbant des charbons actifs sous forme de granulés ou d'extrudés.

Les charbons actifs utilisés ont de grande surface spécifique (>1100 m²/g) et proviennent des sociétés Norit, Ceca et Eurocarb.

On reproduit l'exemple 1 à la seule différence que la nature de l'adsorbant est changée.

Les résultats obtenus au bout de 30 min sont indiqués dans le tableau (I).

**Tableau (I)**

| Charbon actif utilisé | Origine | Surface spécifique | Sélectivité | % gaïacolate adsorbé par rapport au poids total de gaïacolate |
|---|---|---|---|---|
| Norit ROX 8 | Houille | 1225 | 1 | 70 |
| Acticarbone BGX260 | Pin | 1700 | 1 | 76 |
| Chemviron CAL | Noix de coco | 1300 | 1 | 81 |

### Exemple 5

Dans cet exemple, on met en oeuvre le charbon actif Eurocarb HT5 qui est d'origine de noix de coco. Il se présente sous forme de grains et possède une surface spécifique de 1400 m²/g.

Le charbon est mis en oeuvre dans une colonne de 300 mL, d'un diamètre de 2,8 cm et d'une hauteur de 24 cm.

Le volume de charbon est de 150 mL.

La colonne est maintenue sous pression atmosphérique à 35°C.

Sur ce charbon, on fait percoler à une vitesse de 1,5 m/h, un flux comprenant 4,4 % de gaïacolate de sodium et 6,6 % de composés mandéliques sous forme de sel de sodium (o-, p- et dimandélate) issus d'une réaction de condensation entre l'acide glyoxylique et le gaïacol, en présence de soude conduite selon l'enseignement de l'état de la technique (WO 99/65853) et qui répondent aux formules (IIa), (IIb) et (IIc) dans lesquelles R représente un groupe méthoxy, x est égal à 1 et M est le sodium.

Dans cet exemple, on percole sur le charbon 540 g du flux tel que défini précédemment.

On récupère à la sortie de la colonne, 340 g d'un flux exempt de gaïacolate de sodium et contenant la totalité des composés mandéliques sous forme de sels de sodium chargés.

Le rapport qui définit la sélectivité est de 1.

On récupère le gaïacolate de sodium adsorbé sur le charbon par traitement à l'aide de soude.

Une solution aqueuse de soude à 2 % en poids est percolée à travers le charbon à une vitesse de 2 m/s.

On recueille un flux contenant le gaïacolate de sodium avec un rendement de 60 % en poids, le rendement étant défini comme le rapport pondéral (en %) entre le gaïacolate récupéré et le gaïacolate engagé.

Celui-ci peut être directement recyclé à l'étape de condensation.

Le flux en sortie de colonne qui contient les composés mandéliques sous forme salifiée est ensuite oxydé sans ajout supplémentaire de solution aqueuse de soude.

Le flux est chargé dans un réacteur en inox 316L équipé d'une agitation mécanique, de contre-pâles et d'une arrivée d'air.

A ce milieu réactionnel est ajouté un système catalytique comprenant CoCl₂, 6H₂O et CuSO₄, 5H₂O mis en oeuvre respectivement en une quantité exprimée en pourcentage molaire de composés mandéliques de 0,125 et 0,125.

Le milieu est ensuite chauffé jusqu'à 80°C et l'air est introduit à un débit de 1,6 L/h.

Au bout de 30 min de réaction, on obtient une sélectivité de la réaction en vanilline (exprimée par le rapport entre le nombre de moles de vanilline formées et le nombre de moles de p-mandélate transformées) de 98 %.

### Exemple 6

On reproduit l'exemple 1 à la différence près que le milieu réactionnel engagé résulte de la réaction du guétol et de l'acide glyoxylique, en présence de soude et comprend donc du guétolate de sodium et les composés mandéliques sous forme de sels de sodium (o-, p- et dimandélate) qui répondent aux formules (IIa), (IIb) et (IIc) dans lesquelles R représente un groupe éthoxy, x est égal à 1 et M est le sodium.

L'adsorbant utilisé dans cet exemple est le charbon actif Eurocarb HT5.

On obtient après 1 h 30 d'agitation, un milieu contenant la totalité des composés mandéliques sous forme de sels de sodium et 31 % de guétolate de sodium.

Avec ce support adsorbant, le rapport qui définit la sélectivité est de 1.

## Revendications

1. Procédé de séparation de composés phénoliques sous forme salifiée à partir d'un milieu réactionnel aqueux résultant de la réaction d'un composé phénolique et de l'acide glyoxylique en présence d'une base conduisant à un milieu réactionnel comprenant au moins l'excès de composé phénolique de départ sous forme salifiée et les différents composés mandéliques sous forme salifiée résultant de la réaction, **caractérisé par le fait que** l'on met en contact ledit milieu réactionnel comprenant le composé phénolique de départ sous forme salifiée avec un support adsorbant choisi parmi les charbons actifs, les polymères adsorbants de type polystyrène, de type polyacrylique ou de type phénolique, les zéolithes et les tamis moléculaires, conduisant à l'adsorption sélective dudit composé phénolique sur ledit support et à la récupération d'un flux aqueux comprenant les composés mandéliques sous forme salifiée issus de la réaction et que l'on désorbe le composé phénolique fixé sur l'adsorbant, par un traitement de régénération dudit adsorbant.

2. Procédé selon la revendication 1 **caractérisé par le fait que** le milieu réactionnel comprend un composé phénolique sous forme salifiée répondant à la formule suivante : 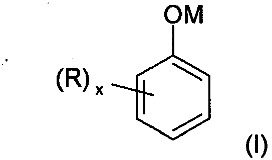 dans ladite formule :
- R est un groupe alkyle ou alkoxy ayant de 1 à 4 atomes de carbone, un atome d'halogène,
- x est un nombre allant de 0 à 3, et plus préférentiellement égal à 1,
- M représente un cation d'un élément métallique du groupe (IA) de la classification périodique à savoir le lithium, sodium, potassium, rubidium et césium ou un cation ammonium.

3. Procédé selon l'une des revendications 1 et 2 **caractérisé par le fait que** le composé phénolique sous forme salifiée de formule (I) est le gaïacolate de sodium ou le guétolate de sodium.

4. Procédé selon l'une des revendications 1 à 3 **caractérisé par le fait que** le milieu réactionnel comprend le composé phénolique sous forme salifiée à une concentration comprise entre 1 et 20 % en poids, de préférence entre 5 et 10 % en poids.

5. Procédé selon l'une des revendications 1 à 4 **caractérisé par le fait que** le milieu réactionnel comprend des produits de réaction qui sont des acides mandéliques sous forme salifiée à une concentration comprise entre 3 % et 30 % en poids, de préférence entre 5 % et 20 % en poids.

6. Procédé selon l'une des revendications 1 à 5 **caractérisé par le fait que** le milieu réactionnel est un milieu aqueux comprenant du gaïacolate de sodium et des composés mandéliques : acide 4-hydroxy-3-méthoxymandélique, acide 2-hydroxy-3-méthoxymandélique, acide 4-hydroxy-5-méthoxy-1,3-dimandélique.

7. Procédé selon l'une des revendications 1 à 6 **caractérisé par le fait que** l'adsorbant est un charbon actif à base de houille, de tourbe, de lignite, de résidus de distillation du pétrole, ou à partir de toute matière organique végétale riche en carbone: bois, écorces, brindilles, pâte de bois, coques de fruits de préférence coques de noix de coco, coques de cacahuètes.

8. Procédé selon l'une des revendications 1 à 6 **caractérisé par le fait que** l'adsorbant est un adsorbant carboné qui résulte de la pyrolyse d'une résine polymère.

9. Procédé selon l'une des revendications 1 à 6 **caractérisé par le fait que** l'adsorbant est un polymère adsorbant à structure poreuse.

10. Procédé selon l'une des revendications 1 à 6 **caractérisé par le fait que** le polymère adsorbant de type polystyrène est un copolymère styrène-divinylbenzène, que le polymère adsorbant de type polyacrylique est un copolymère divinylbenzène - ester acrylique et que le polymère adsorbant de type phénolique est un copolymère phénol/formaldéhyde.

11. Procédé selon l'une des revendications 1 à 6 **caractérisé par le fait que** l'adsorbant est une zéolithe ayant un diamètre moyen de pores supérieur ou égal à 6 , et de préférence compris entre 6 et 8 Å.

12. Procédé selon l'une des revendications 1 à 6 et la revendication 11 **caractérisé par le fait que** l'adsorbant est une zéolithe de type mordénite ; une zéolithe de type β, une zéolithe de type faujasite, de préférence une zéolithe Y ; une zéolithe mésoporeuse de type MCM.

13. Procédé selon l'une des revendications 1 à 6 **caractérisé par le fait que** l'adsorbant est un tamis moléculaire, de préférence un tamis moléculaire 5A.

14. Procédé selon l'une des revendications 1 à 13 **caractérisé par le fait que** la quantité de support adsorbant mis en oeuvre représente au moins de 2 à 10 fois le poids du composé phénolique sous forme salifiée à adsorber.

15. Procédé selon l'une des revendications 1 à 14 **caractérisé par le fait que** l'on récupère le composé phénolique sous forme salifiée, par régénération de l'adsorbant par un traitement basique, de préférence à l'aide d'une solution aqueuse de soude.

## Patentansprüche

1. Verfahren zum Abtrennen von Phenolverbindungen in versalzter Form aus einem wässrigen Reaktionsmedium, das bei der Umsetzung einer Phenolverbindung mit Glyoxylsäure in Gegenwart einer Base anfällt, die zu einem Reaktionsmedium führt, das mindestens den Überschuss der Ausgangs-Phenolverbindung in versalzter Form und die verschiedenen, sich durch die Umsetzung ergebenden Mandelsäure-Verbindungen in versalzter Form umfasst, **dadurch gekennzeichnet, dass** man das Reaktionsmedium, das die Ausgangs-Phenolverbindung in versalzter Form umfasst, mit einem adsorbierenden Träger, der aus Aktivkohlen, adsorbierenden Polymeren vom Polystyrol-Typ, Polyacryl-Typ oder Phenol-Typ, Zeolithen und Molsieben ausgewählt ist, in Berührung bringt, was zur selektiven Adsorption der Phenolverbindung an dem Träger und zur Gewinnung eines wässrigen Stroms, der die aus der Umsetzung stammenden Mandelsäure-Verbindungen in versalzter Form umfasst, führt, und die an dem Adsorptionsmittel fixierte Phenolverbindung durch eine Regenerationsbehandlung des Adsorptionsmittels desorbiert.

2. Verfahren nach Anspruch 1, **dadurch gekennzeichnet, dass** das Reaktionsmedium eine Phenolverbindung in versalzter Form umfasst, die folgender Formel entspricht: 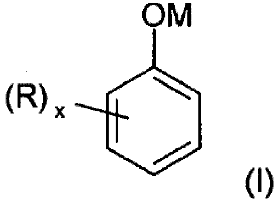 wobei in der Formel:
- R für eine Alkyl- oder Alkoxygruppe mit 1 bis 4 Kohlenstoffatomen oder ein Halogenatom steht,
- x für eine Zahl von 0 bis 3 steht und weiter bevorzugt gleich 1 ist,
- M für ein Kation eines Metallelements der Gruppe (IA) des Periodensystems, nämlich Lithium, Natrium, Kalium, Rubidium und Caesium, oder ein Ammoniumkation steht.

3. Verfahren nach einem der Ansprüche 1 und 2, **dadurch gekennzeichnet, dass** es sich bei der Phenolverbindung in versalzter Form der Formel (I) um Natriumguajacolat oder Natriumguaetholat handelt.

4. Verfahren nach einem der Ansprüche 1 bis 3, **dadurch gekennzeichnet, dass** das Reaktionsmedium die Phenolverbindung in versalzter Form in einer Konzentration zwischen 1 und 20 Gew.-%, vorzugsweise zwischen 5 und 10 Gew.-%, umfasst.

5. Verfahren nach einem der Ansprüche 1 bis 4, **dadurch gekennzeichnet, dass** das Reaktionsmedium Reaktionsprodukte, bei denen es sich um Mandelsäuren in versalzter Form handelt, in einer Konzentration zwischen 3 und 30 Gew.-%, vorzugsweise zwischen 5 und 20 Gew.-%, umfasst.

6. Verfahren nach einem der Ansprüche 1 bis 5, **dadurch gekennzeichnet, dass** es sich bei dem Reaktionsmedium um ein wässriges Medium handelt, das Natriumguajacolat und Mandelsäure-Verbindungen: 4-Hydroxy-3-methoxymandelsäure, 2-Hydroxy-3-methoxymandelsäure, 4-Hydroxy-5-methoxy-1,3-dimandelsäure umfasst.

7. Verfahren nach einem der Ansprüche 1 bis 6, **dadurch gekennzeichnet, dass** es sich bei dem Adsorptionsmittel um eine Aktivkohle auf Basis von Kohle, Torf, Lignit oder Erdöldestillationsrückständen oder auf Basis eines kohlenstoffreichen pflanzlichen organischen Materials: Holz, Rinden, Reisig, Zellstoff, Fruchtschalen, vorzugsweise Kokosnussschalen und Erdnussschalen, handelt.

8. Verfahren nach einem der Ansprüche 1 bis 6, **dadurch gekennzeichnet, dass** es sich bei dem Adsorptionsmittel um ein kohlenstoffhaltiges Adsorptionsmittel, das sich aus der Pyrolyse eines Polymerharzes ergibt, handelt.

9. Verfahren nach einem der Ansprüche 1 bis 6, **dadurch gekennzeichnet, dass** es sich bei dem Adsorptionsmittel um ein adsorbierendes Polymer mit poröser Struktur handelt.

10. Verfahren nach einem der Ansprüche 1 bis 6, **dadurch gekennzeichnet, dass** es sich bei dem adsorbierenden Polymer vom Polystyrol-Typ um ein Styrol-Divinylbenzol-Copolymer handelt, es sich bei dem adsorbierenden Polymer vom Polyacryl-Typ um ein Divinylbenzol-acrylsäureester-Copolymer handelt und es sich bei dem adsorbierenden Polymer vom Phenol-Typ um ein Phenol/Formaldehyd-Copolymer handelt.

11. Verfahren nach einem der Ansprüche 1 bis 6, **dadurch gekennzeichnet, dass** es sich bei dem Adsorptionsmittel um einen Zeolith mit einem mittleren Porendurchmesser größer gleich 6 und vorzugsweise zwischen 6 und 8 Å handelt.

12. Verfahren nach einem der Ansprüche 1 bis 6 und Anspruch 11, **dadurch gekennzeichnet, dass** es sich bei dem Adsorptionsmittel um einen Zeolith vom Mordenit-Typ; einen Zeolith vom β-Typ; einen Zeolith vom Faujasit-Typ, vorzugsweise einen Y-Zeolith oder einen mesoporösen Zeolith vom MCM-Typ handelt.

13. Verfahren nach einem der Ansprüche 1 bis 6, **dadurch gekennzeichnet, dass** es sich bei dem Adsorptionsmittel um ein Molsieb, vorzugsweise ein 5A-Molsieb handelt.

14. Verfahren nach einem der Ansprüche 1 bis 13, **dadurch gekennzeichnet, dass** die eingesetzte Menge des adsorbierenden Trägers mindestens das 2- bis 10-fache des Gewichts der zu adsorbierenden Phenolverbindung in versalzter Form beträgt.

15. Verfahren nach einem der Ansprüche 1 bis 14, **dadurch gekennzeichnet, dass** die Phenolverbindung in versalzter Form durch Regeneration des Adsorptionsmittels durch basische Behandlung, vorzugsweise mit Hilfe einer wässrigen Natriumhydroxidlösung, zurückgewonnen wird.

## Claims

1. Method of separating phenolic compounds in salified form from an aqueous reaction mixture resulting from the reaction of a phenolic compound and glyoxylic acid in the presence of a base, leading to a reaction mixture comprising at least the excess of phenolic starting compound in salified form and the various mandelic compounds in salified form, resulting from the reaction, **characterized in that** said reaction mixture comprising the phenolic starting compound in salified form is contacted with an adsorbent support selected from activated carbons, adsorbent polystyrene, polyacrylic or phenolic polymers, zeolites, and molecular sieves, leading to the selective adsorption of said phenolic compound on said support, and to the recovery of an aqueous stream comprising the mandelic compounds in salified form obtained from the reaction, and **in that** the phenolic compound fixed on the adsorbent is desorbed by a regenerative treatment of said adsorbent.

2. Method according to Claim 1, **characterized in that** the reaction mixture comprises a phenolic compound in salified form corresponding to the following formula: 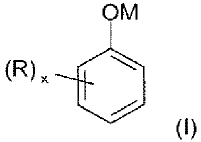 in which formula:
- R is an alkyl or alkoxy group having from 1 to 4 carbon atoms, or a halogen atom,
- x is a number from 0 to 3, and more preferably is 1, and
- M represents a cation of a metallic element from group (IA) of the periodic table, namely lithium, sodium, potassium, rubidium, and cesium, or an ammonium cation.

3. Method according to either of Claims 1 and 2, **characterized in that** the phenolic compound in salified form of formula (I) is sodium guaiacolate or sodium guaetholate.

4. Method according to any of Claims 1 to 3, **characterized in that** the reaction mixture comprises the phenolic compound in salified form at a concentration of between 1% and 20% by weight, preferably between 5% and 10% by weight.

5. Method according to any of Claims 1 to 4, **characterized in that** the reaction mixture comprises reaction products which are mandelic acids in salified form at a concentration of between 3% and 30% by weight, preferably between 5% and 20% by weight.

6. Method according to any of Claims 1 to 5, **characterized in that** the reaction mixture is an aqueous mixture comprising sodium guaiacolate and mandelic compounds: 4-hydroxy-3-methoxymandelic acid, 2-hydroxy-3-methoxymandelic acid, 4-hydroxy-5-methoxy-1,3-dimandelic acid.

7. Method according to any of Claims 1 to 6, **characterized in that** the adsorbent is an activated carbon based on coal, peat, lignite, or petroleum distillation residues, or on the basis of any carbon-rich organic vegetable matter: wood, barks, twigs, wood pulp, shells of fruits, preferably coconut shells and groundnut shells.

8. Method according to any of Claims 1 to 6, **characterized in that** the adsorbent is a carbon-containing adsorbent resulting from the pyrolysis of a polymeric resin.

9. Method according to any of Claims 1 to 6, **characterized in that** the adsorbent is an adsorbent polymer having a porous structure.

10. Method according to any of Claims 1 to 6, **characterized in that** the adsorbent polystyrene polymer is a styrene-divinylbenzene copolymer, the adsorbent polyacrylic polymer is a divinylbenzene-acrylic ester copolymer and the adsorbent phenolic polymer is a phenol/formaldehyde copolymer.

11. Method according to any of Claims 1 to 6, **characterized in that** the adsorbent is a zeolite having an average pore diameter of greater than or equal to 6, and preferably of between 6 and 8 Å.

12. Method according to any of Claims 1 to 6 and Claim 11, **characterized in that** the adsorbent is a mordenite zeolite; a β zeolite, a faujasite zeolite, preferably a Y zeolite; or a mesoporous MCM zeolite.

13. Method according to any of Claims 1 to 6, **characterized in that** the adsorbent is a molecular sieve, preferably a 5A molecular sieve.

14. Method according to any of Claims 1 to 13, **characterized in that** the amount of adsorbent support employed represents at least from 2 to 10 times the weight of the phenolic compound in salified form that is to be adsorbed.

15. Method according to any of Claims 1 to 14, **characterized in that** the phenolic compound in salified form is recovered by regeneration of the adsorbent by a basic treatment, preferably by means of an aqueous sodium hydroxide solution.
